# EUROPEAN PATENT SPECIFICATION

(11) **EP 4 284 274 B1**
(45) Date of publication and mention of the grant of the patent: **28.10.2026**
(21) Application number: 22705238.8
(22) Date of filing: 28.01.2022
(51) Int. Cl.: A61B 17/92

(54) **ORTHOPEDIC IMPACTOR TOOL**
ORTHOPÄDISCHES IMPAKTORWERKZEUG
OUTIL PERCUTEUR ORTHOPÉDIQUE

(30) Priority: 29.01.2021 US 202163143548 P
(43) Date of publication of application: 06.12.2023
(73) Proprietor: Zimmer, Inc., Warsaw, Indiana 46580 (US)
(72) Inventor: MARINKOVICH, Dragomir, Hale Corners, Wisconsin 53130 (US)
(74) Representative: Taor, Simon Edward William
(86) International application number: PCT/US2022/014380
(87) International publication number: WO 2022/165223

(56) References cited:
- US-A1- 2012 172 939
- US-A1- 2012 215 267

## Description

### FIELD OF THE DISCLOSURE

The present disclosure relates generally to surgical instruments. More specifically, the present disclosure relates to an orthopedic impactor tool.

### BACKGROUND

Orthopedic surgeons commonly utilize tools for cutting or carving bone that require a hammer or mallet to transmit an impaction force to the tool. An example is a broach tool used to prepare the proximal end of a femur to receive the stem of a hip implant. Such broaches can be used with a hammer wielded by the physician or with a pneumatic "jackhammer" like tool. However, striking a broach tool with a hammer can be tiresome and can cause high stresses on the physician's own joints, such as the shoulder joint. Furthermore, pneumatic impact tools require connection to an air hose, which can be inconvenient and can potentially limit the physician's ability to orient the tool in the desired manner.

US2012215267A1 disclsoes an orthopedic impacting tool comprising a motor, a linear motion converter, an air chamber, a compression piston, a striker, a detent and a broach adapter. The compression piston may cause the striker to apply controlled force on a adapter to create a precise opening for subsequently disposing a prosthesis in a patient. The detent may hold the striker in a position until a sufficient pressure is applied to release the striker. The tool allows forward or backward impacting for expanding the size or volume of the opening or for facilitating removal of the broach and tool from the opening. A force adjustment control of the tool allows a user to increase or decrease the impact force. A light source and hand grips improve ease of operation of the tool.

### SUMMARY

The following, non-limiting examples, detail certain aspects of the present subject matter to solve the challenges and provide the benefits discussed herein, among others.

According to an aspect, there is provided an orthopedic impact tool as set out in claim 1. Optional features are set out in the dependent claims.

### BRIEF DESCRIPTION OF THE FIGURES

In the drawings, which are not necessarily drawn to scale, like numerals can describe similar components in different views. Like numerals having different letter suffixes can represent different instances of similar components. The drawings illustrate generally, by way of example, but not by way of limitation, various embodiments discussed in the present document.
FIG. 1 shows a side plan view of an orthopedic impact tool consistent with at least one example of this disclosure.
FIG. 2 shows a cross-sectional view of an orthopedic impact tool consistent with at least one example of this disclosure.
FIGS. 3A, 3B, and 3C shows plan views of a three position trigger consistent with at least one example of this disclosure.
FIG. 4 shows a cross-sectional view of an impact mechanism of an orthopedic impact tool consistent with at least one example of this disclosure.
FIG. 5 shows a cross-sectional view of an impact mechanism of an orthopedic impact tool consistent with at least one example of this disclosure.
FIG. 6 shows a cross-sectional view of an impact mechanism of an orthopedic impact tool shown consistent with at least one example of this disclosure.
FIG. 7 shows a cross-sectional view of an impact mechanism of an orthopedic impact tool consistent with at least one example of this disclosure.
FIG. 8 shows a cross-sectional view of an impact mechanism of an orthopedic impact tool consistent with at least one example of this disclosure.
FIG. 9 shows a cross-sectional view of an impact mechanism of an orthopedic impact tool consistent with at least one example of this disclosure.
FIG. 10 shows a cross-sectional view of an impact mechanism of an orthopedic impact tool consistent with at least one example of this disclosure.
FIG. 11 shows a cross-sectional view of an impact mechanism of an orthopedic impact tool consistent with at least one example of this disclosure.
FIG. 12 shows a cross-sectional view of an impact mechanism of an orthopedic impact tool consistent with at least one example of this disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

As an alternative to a pneumatic piston driven system, disclosed herein are electrically driven systems. Specifically, the orthopedic impact tools disclosed herein can include a housing, a tube assembly, a reverse impact plate, an anvil and an impact mechanism. The housing can include a hand grip portion and an impact mechanism housing portion. The tube assembly can include a first tube portion and a second tube portion. The reverse impact plate can be disposed in between the first tube portion and the second tube portion. The anvil can include a forward impact surface. The impact mechanism can include a motor, a piston, a rain, and a rotary to linear conversion mechanism drivingly connecting the motor to the piston. The piston can be disposed within the first tube portion and the ram can be disposed in the second tube portion and movable into contact between the reverse impact plate and the forward impact surface.

The tube assembly, the anvil, and the reverse impact plate can be axially shiftable rearward from a neutral position within the housing for activating a forward impact cycle. The tube assembly, the anvil, and the reverse impact plate can be axially shiftable forward from the neutral position within the housing for activating a rearward impact cycle.

As part of developing a reciprocating motion, the tube assembly can define a chamber port that is open to an atmosphere when the tube assembly is in a neutral position and closed to the atmosphere when the tube assembly is shifted rearward from the neutral position. During motion, air can be drawn into the tube assembly to create pressure used to drive elements, such as the ram, the generate impact forces.

The orthopedic impact tools can be controlled by an energy level switch and a three position trigger switch. A control unit can be operative to control the motor based upon a position of the energy level switch to change a magnitude of impact forces delivered. The three position trigger switch can include an off position, a single operation position, and a continuous operation position. When in the single operation position the orthopedic impact tools can deliver a single impact. When in the continuous position, the orthopedic impact tools can deliver continuous impacts.

The above discussion is intended to provide an overview of subject matter of the present patent application. It is not intended to provide an exclusive or exhaustive explanation of the invention. The description below is included to provide further information about the present patent application.

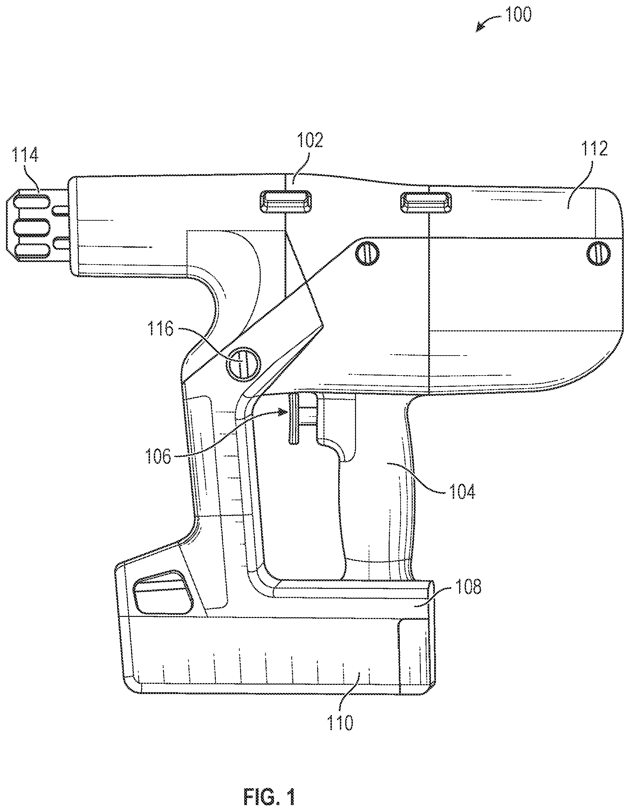

Turning now to the figures, FIG. 1 shows an orthopedic impactor tool 100 consistent with at least one example of this disclosure. The orthopedic impactor tool 100 can include a housing 102 that can define a hand grip portion 104. The hand grip portion 104 can include a trigger 106, a battery support portion 108 that can support a battery pack 110 and an impact mechanism housing portion 112. The impact mechanism housing portion 112 can support an impact mechanism 228 therein that can deliver impacts to a chuck 114.

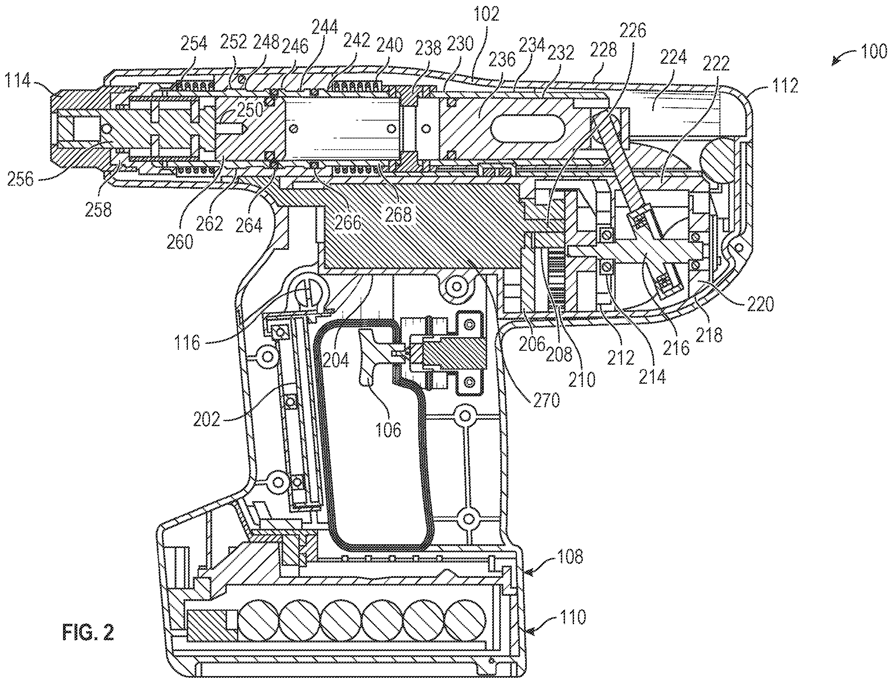

With reference to FIG. 2, a sectional view of the orthopedic impactor tool 100 is shown including details of the impact mechanism 228 disposed within the impact mechanism housing portion 112. As shown in FIG. 2, the impact mechanism 228 can include a motor 270 that can include a drive shaft 226. The drive shaft 226 can be connected to a drive gear 208. The drive gear 208 can be engaged with a driven gear wheel 210 that can have internal gear teeth. The driven gear wheel 210 can be connected to a swashplate 216 or other rotary to linear conversion device. For example, the swashplate 216 can be a crank arm. The swashplate 216 or other rotary to linear conversion device can be connected to a piston 236 that can be disposed within a rear portion 232 of a tube assembly 40. The tube assembly 230 can include a forward portion 268 that can be separated from the rear portion 232 by a reverse impact plate 238.

A ram 260 can be disposed in the forward portion 268 of the tube assembly 230 and can be movable between a forward position for impacting a forward impact surface 250 of an anvil 256 and a rearward position for impacting the reverse impact plate 238. The chuck 114 can be connected to a forward end of the anvil 256. The chuck 114 can be adapted to engage a broach or other tool (not shown) that can be utilized for creating a cavity within a bone for receiving a prosthetic device. When the ram 260 contacts the impact surface 250 of the anvil 256, impact energy can be transferred through the proximal impact surface 250 to the implement. When the ram 260 contacts the reverse impact plate 238, impact energy can be transferred through the tube assembly 230 and the chuck 114 to the implement.

The housing 102 can define a hollow cavity 224 including an integrally formed internal wall structure 204 that can support the motor 270, the trigger 106 and a controller unit 202. A frame 222 can be further received within the cavity 224 and can include a first support portion 206 for supporting the drive shaft 226 and second and third support portions 212 and 218 for supporting forward and rearward swashplate support bearings 214 and 220.

The frame 222 can further include a first tube assembly support portion 234 that can support the rear portion 232 of the tube assembly 230. The frame 222 can also include a second tube assembly support portion 246 that can support the forward portion 268 of the tube assembly 230. The tube assembly can slide within the housing 102 and can be guided by the first tube assembly support portion 234 and the second tube assembly support portion 246. The second tube assembly support portion 246 can further include a plurality of annular grooves 262, 264, and 266 disposed in an inner surface and defined by a plurality of land portions 242, 244, 248, and 252.

A first spring 254 can be disposed between a forward end of the tube assembly support portion 246 of the frame 222 and a nose cap 258 of the forward portion 268 of the tube assembly 230. A second spring 240 can be disposed between a rear end of the tube assembly support portion 246 of the frame 222 and an outer annular body 902 of the reverse impact plate 238.

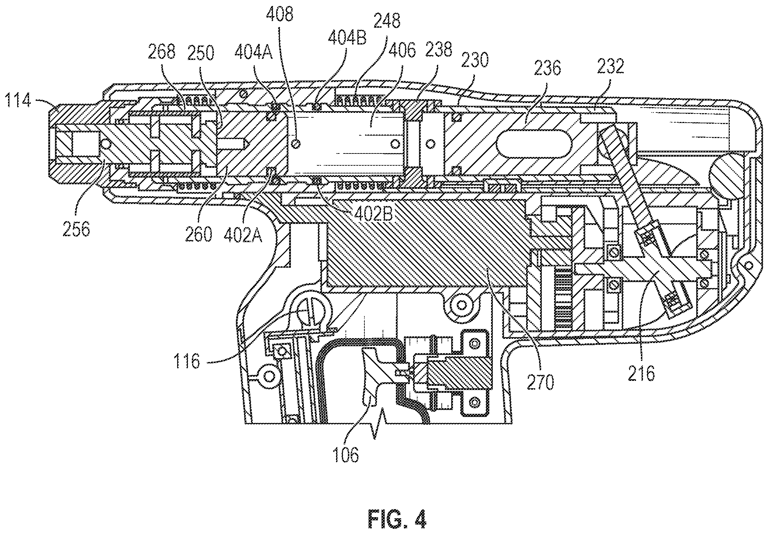

With reference to FIG. 4, the forward portion 268 of the tube assembly 230 can include a pair of recessed grooves 404 (labeled individually as grooves 404A and 404B) each supporting an O-ring 402A and 402B therein. As shown in FIG. 4, the forward portion 268 of the tube assembly 230 can include a chamber 406 defining a chamber port 408 that can be in communication with the atmosphere when in a neutral state or closed off from the atmosphere during forward and reverse impact operations, as disclosed herein.

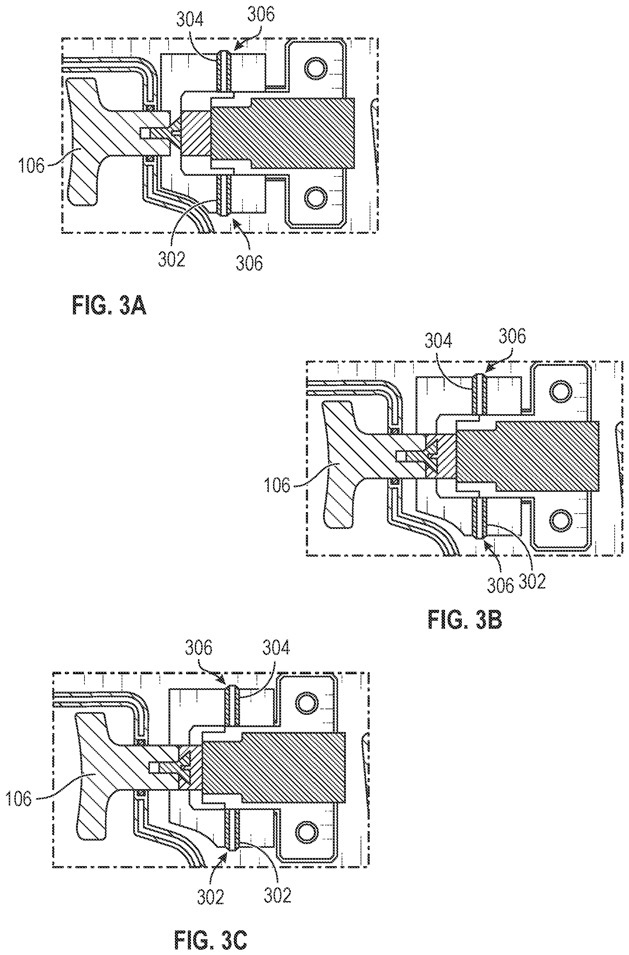

As shown in FIGS. 3A, 3B, and 3C, the trigger 106 can have an off position (FIG. 3A) and can be depressed to two different depths. In a first depressed position, as shown in FIG. 3B, two spring pins 304 can reach a pair of detents at 306. In a second further depressed position, the spring pins 304 move past the detents at 306. Depressing the trigger 106 to the first depth can cause a single impact of the impact mechanism 228 to be delivered. Further depressing the trigger 106 to the second depth can cause impacts of the impact mechanism 228 to be delivered continuously until the trigger 106 is released.

The tool 100 can further include an impact energy switch 116. The impact energy switch 116 can be a rotary switch that can provide an input to the controller unit 202. In the first position of the impact energy switch 116, the controller unit 202 can cause the impact mechanism 228 to deliver low-energy impacts. In the second position of the impact energy switch 116, the controller unit 202 can cause the impact mechanism 228 to deliver high-energy impacts as disclosed herein.

The chuck 114, the anvil 256, the reverse impact plate 238, the ram 260 and the tube assembly 230 can be "shifting components" that can translate with respect to the rest of the tool 100 along the axis of the tube assembly 230. With reference to FIG. 4, a cross sectional view of the orthopedic impact tool 100 is shown in a neutral state. In the neutral state, the ram 260 and piston 236 are in a forward position. In addition, the tube assembly 230 is positioned by the first and second springs 254, 240 such that the chamber port 406 is open to the atmosphere so movement of the piston 236 does not create compression within the tube assembly 230.

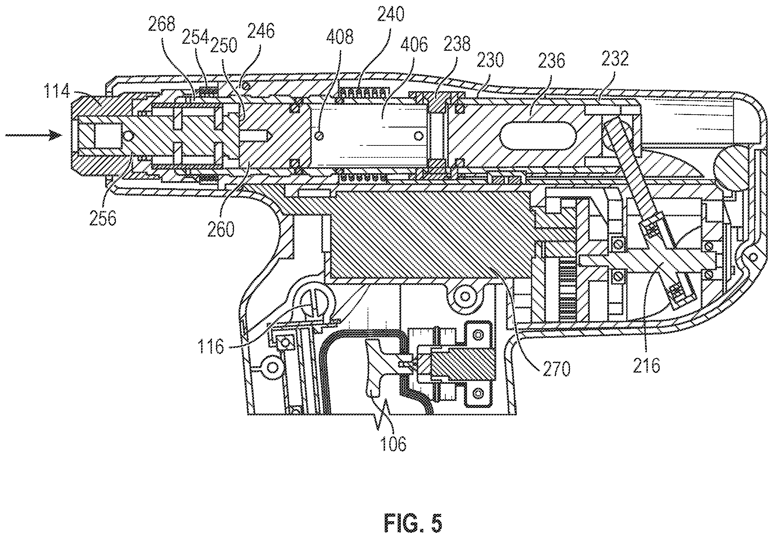

In order to activate a forward impact cycle, an implement held by the chuck 114 of the orthopedic tool 100 can be pressed against a bone and the shifting components 114, 230, 238, 260, 256 can be pressed rearward so that the spring 254 is in a compressed state, as shown in FIG. 5, so that the chamber port 406 is sealed and the pressure chamber 302 is closed. In particular, the O-rings 402A and 402B can sealingly engage the lands 244, 242 on opposite sides of groove 266, closing off the port 406 from atmosphere. The closing of the pressure chamber 302 allows high and low pressures to form within the tube assembly 230 so that reciprocal movement of the piston 236 can cause the ram 260 to reciprocate and contact the impact surface 250 of the anvil 256.

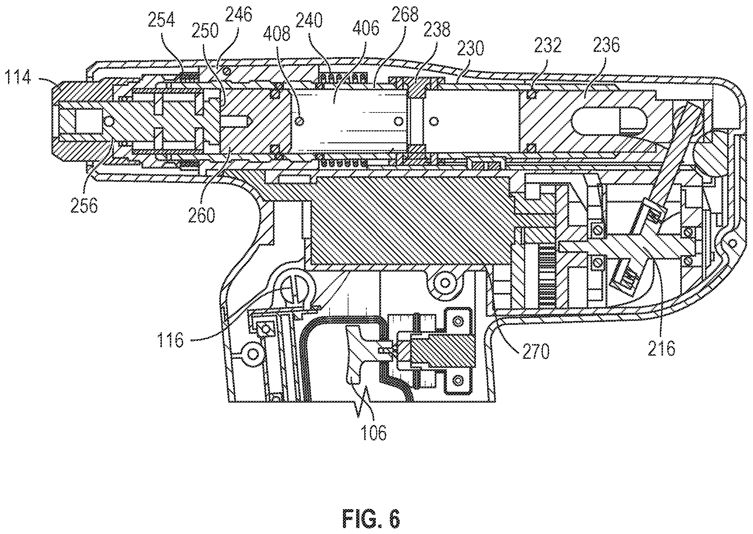

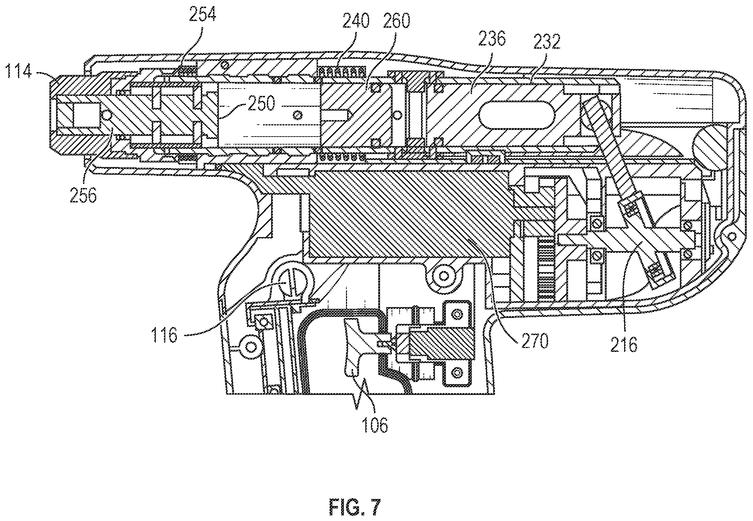

To begin, the controller unit 202 can respond to the activation of the trigger 106 to instruct the motor 270 to begin rotating. The motor 270 can turn the gears 208, 210 and can rotate the swashplate 216 as well. The rotation of the swashplate 216 can cause it to pull the piston 236 rearward, as shown in FIG. 6. This can create a low pressure region in the pressure chamber 302 that can draw the ram 260 rearward. The motor 270 and swashplate 216 can continue to rotate, causing the piston 236 to be moved forward. This can create a high pressure region in the pressure chamber 302 as shown in FIG. 7.

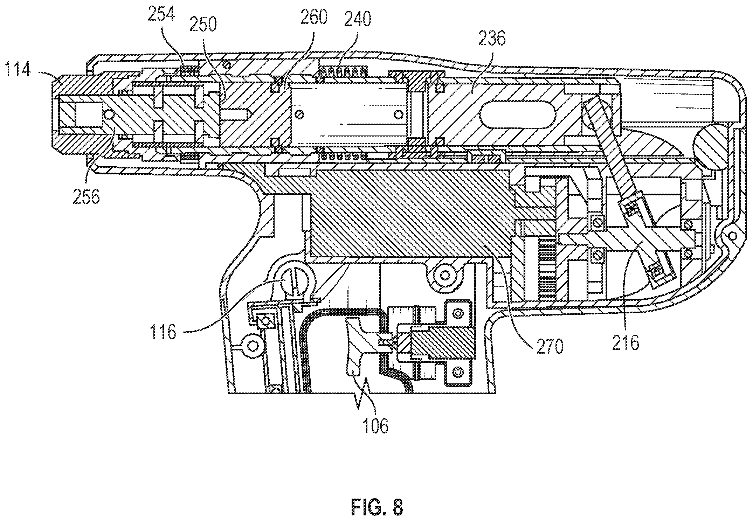

The high pressure region can push the ram 260 proximally until it contacts the forward impact surface 250 of the anvil 256, as shown in FIG. 8. The kinetic energy of the ram 260 can be transferred through the anvil 256 to the orthopedic implement. The rearward shifting of the shifting components 114, 230, 238, 260, 256 can prevent the ram 260 from contacting the rearward impact plate 238 during the forward impact cycle.

The speed at which the motor 270 and swashplate 216 rotate can determine the impact energy of the ram 260 and the frequency of the impacts. The speed can be modified by the controller unit 202 when the impact energy switch 116 is rotated.

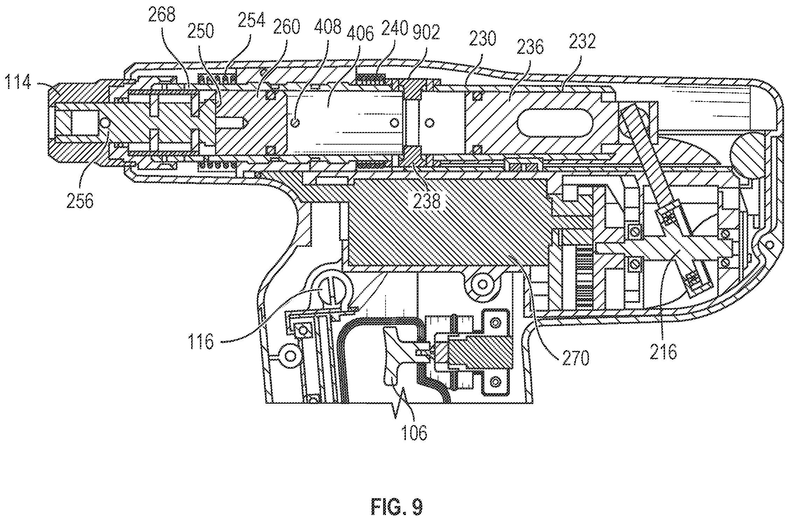

In order to activate a rearward impact cycle, the device can start in a neutral state, as shown in FIG. 4, where again, the pressure chamber 302 is open to atmosphere via the chamber port 406. Before impacting, an external pulling force can be applied to the orthopedic impact tool 100 to compress the spring 240 putting the device in an extended state as shown in FIG. 9. In the extended state as shown in FIG. 9, the shifting components 114, 230, 238, 260, 256 can be pulled forward so that the chamber port 406 can be sealed off and the pressure chamber 302 can be closed to allow high and low pressures to form within the pressure chamber 302. Sealing of the chamber port 406 can occur when the O-rings 402A and 402B engage the lands 248, 244 and closes off the groove 264 from atmosphere.

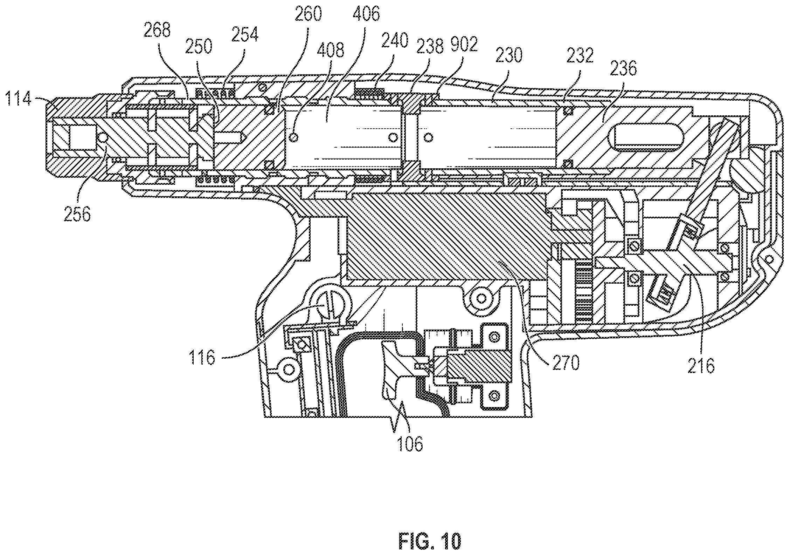

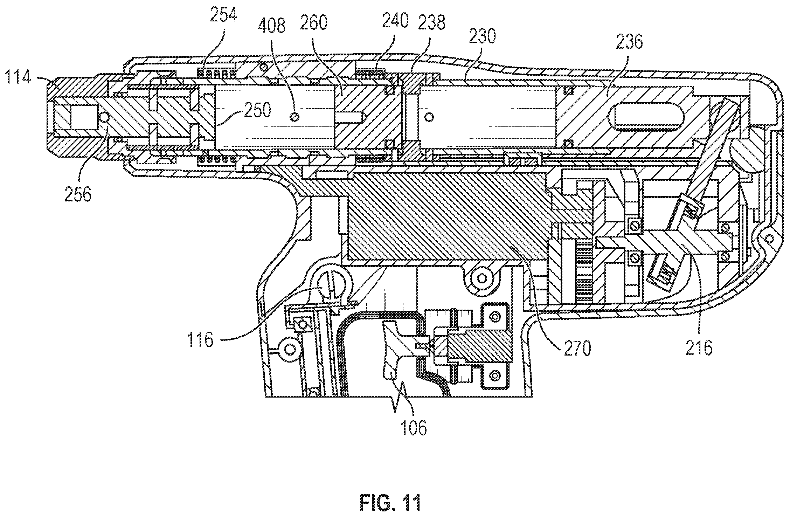

To begin the rearward impact cycle, the controller unit 202 can respond to the depression of the trigger 106 and instruct the motor 270 to begin rotating (e.g., by transmitting a signal, such as a voltage, to the motor 270). The motor 270 can turn the gears 208, 210 and rotate the swashplate 216 as well. The rotation of the swashplate 216 can cause it to pull the piston 236 rearward, as shown in FIG. 10. This can create a low pressure region in the pressure chamber 302 that can draw the ram 260 rearward. The low pressure region can pull the ram 260 rearwardly until it contacts the reverse impact plate 238, as shown in FIG. 11. The kinetic energy of the ram 260 can then be transferred through the rearward impact plate 238, the tube assembly 230, and the chuck 114 to the orthopedic implement.

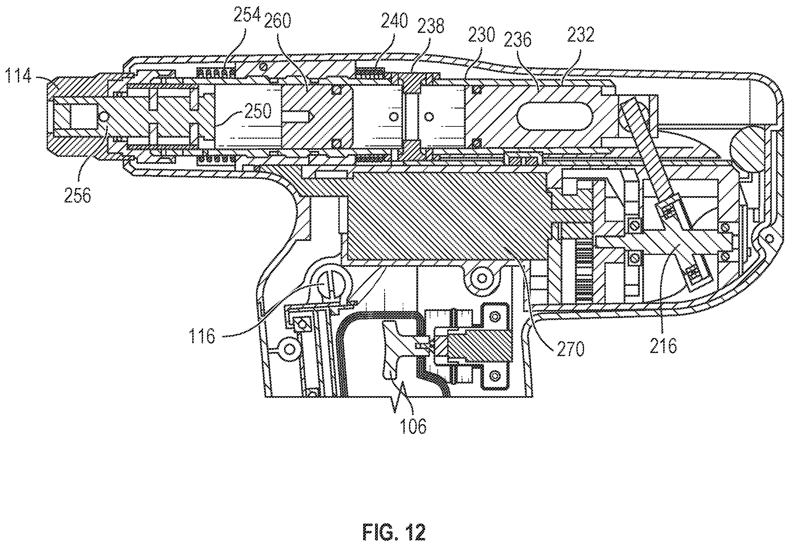

The motor 270 and swashplate 216 can continue to rotate, causing the piston 236 to be moved forward. This can create a high pressure region in the pressure chamber 302 as shown in FIG. 12. The high pressure region can push the ram 260 forward within the tube assembly 230. The forward shifting of the shifting components 114, 230, 238, 260, 256 can prevent the ram 260 from contacting the forward impact surface 250 of the anvil 256 during the rearward impact cycle.

The orthopedic impact tool 100 of the present disclosure can be prevented from delivering impacts when the orthopedic impact tool 100 is in the neutral position. The orthopedic impact tool 100 can only operate when the orthopedic impact tool 100 is being pushed forward toward a bone or pulled rearward away from the bone to provide a simple transition between delivering forward or rearward impacts. The orthopedic impact tool 100 can also provide for multiple impact level choices. The tool can also use a three position trigger switch to switch between single impact and continuous impact mode. The three position trigger switch can allow for more reliable control than a duration system.

### NOTES

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment The scope of the invention is determined by the appended claims.

## Claims

1. An orthopedic impact tool (100) comprising:
a housing (102) including a hand grip portion (104) and an impact mechanism housing portion (112);
a tube assembly (230) comprising a first tube portion and a second tube portion;
a reverse impact plate (238) disposed in between the first tube portion and the second tube portion;
an anvil (256) comprising a forward impact surface (250); and
an impact mechanism (228) comprising:
a motor (270),
a piston (236) disposed within the first tube portion of the tube assembly (230),
a rotary to linear conversion mechanism (216) drivingly connecting the motor (270) to the piston (236),
a ram (260) disposed in the second tube portion and movable into contact between the reverse impact plate (238) and the forward impact surface (250);
wherein the tube assembly (230), the anvil (256), and the reverse impact plate (238) are axially shiftable rearward from a neutral position within the housing (102) for activating a forward impact cycle.

2. The impact tool (100) of claim 1, wherein the tube assembly (230), the anvil (256), and the reverse impact plate (238) are axially shiftable forward from a neutral position within the housing (102) for activating a rearward impact cycle.

3. The orthopedic impact tool (100) of claim 1, wherein the tube assembly (230) defines a chamber port that is:
open to an atmosphere when the tube assembly (230) is in a neutral position; and
closed to the atmosphere when the tube assembly (230) is shifted rearward from the neutral position.

4. The orthopedic impact tool (100) of claim 2, wherein forward shifting of the tube assembly (230) and the anvil (256) prevents the ram (260) from contacting the forward impact surface (250) of the anvil (256) during a rearward impact cycle.

5. The orthopedic impact tool (100) of claim 1, wherein rearward shifting of the tube assembly (230) and the reverse impact plate (238) prevents the ram (260) from contacting the reverse impact plate (238) during a forward impact cycle.

6. The orthopedic impact tool (100) of claim 1, wherein the rotary to linear conversion mechanism includes a swashplate (216).

7. The orthopedic impact tool (100) of any one of or combination of claims 1-6, further comprising a three position trigger switch including an off position, a single operation position, and a continuous operation position.

8. The orthopedic impact tool (100) of claim 7, wherein the three position trigger switch includes the single operation position and the continuous operation position defined by at least one detent of the three position trigger switch.

9. The orthopedic impact tool (100) of any one of or combination of claims 1-6, further comprising:
an energy level switch; and
a control unit operative to control the motor (270) based upon a position of the energy level switch.

10. The orthopedic impact tool (100) of claim 1, further comprising a chuck connected to the anvil (256).

11. The orthopedic impact tool (100) of claim 1, further comprising a frame structure disposed within the housing (102) and supporting the tube assembly (230).

12. The orthopedic impact tool (100) according to claim 11, further comprising a swashplate supported by the frame structure.

## Patentansprüche

1. Orthopädisches Impaktwerkzeug (100), umfassend:
ein Gehäuse (102), das einen Handgriffabschnitt (104) und einen Impaktmechanismusgehäuseabschnitt (112) beinhaltet;
eine Rohrbaugruppe (230), die einen ersten Rohrabschnitt und einen zweiten Rohrabschnitt umfasst;
eine Rückimpaktplatte (238), die zwischen dem ersten Rohrabschnitt und dem zweiten Rohrabschnitt angeordnet ist;
einen Amboss (256), der eine Vorwärtsimpaktfläche (250) umfasst; und
einen Impaktmechanismus (228), umfassend:
einen Motor (270),
einen Kolben (236), der innerhalb des ersten Rohrabschnittes der Rohrbaugruppe (230) angeordnet ist,
einen Dreh-Linear-Umwandlungsmechanismus (216), der den Motor (270) antreibend mit dem Kolben (236) verbindet,
einen Stößel (260), der in dem zweiten Rohrabschnitt angeordnet und in Kontakt zwischen der Rückimpaktplatte (238) und der Vorwärtsimpaktfläche (250) bewegbar ist;
wobei die Rohrbaugruppe (230), der Amboss (256) und die Rückimpaktplatte (238) aus einer neutralen Position innerhalb des Gehäuses (102) axial nach hinten verschiebbar sind, um einen Vorwärtsimpaktzyklus zu aktivieren.

2. Impaktwerkzeug (100) nach Anspruch 1, wobei die Rohrbaugruppe (230), der Amboss (256) und die Rückimpaktplatte (238) aus einer neutralen Position innerhalb des Gehäuses (102) axial vorwärts verschiebbar sind, um einen Rückwärtsimpaktzyklus zu aktivieren.

3. Orthopädisches Impaktwerkzeug (100) nach Anspruch 1, wobei die Rohrbaugruppe (230) eine Kammeröffnung definiert, die wie folgt ist:
offen zu einer Atmosphäre, wenn die Rohrbaugruppe (230) in einer neutralen Position ist;
und
geschlossen zu der Atmosphäre, wenn die Rohrbaugruppe (230) aus der neutralen Position rückwärts verschoben wird.

4. Orthopädisches Impaktwerkzeug (100) nach Anspruch 2, wobei Vorwärtsverschiebung der Rohrbaugruppe (230) und des Ambosses (256) verhindert, dass der Stößel (260) die Vorwärtsimpaktfläche (250) des Ambosses (256) während eines Rückwärtsimpaktzyklus kontaktiert.

5. Orthopädisches Impaktwerkzeug (100) nach Anspruch 1, wobei Rückwärtsverschiebung der Rohrbaugruppe (230) und der Rückimpaktplatte (238) verhindert, dass der Stößel (260) die Rückimpaktplatte (238) während eines Vorwärtsimpaktzyklus kontaktiert.

6. Orthopädisches Impaktwerkzeug (100) nach Anspruch 1, wobei der Dreh-Linear-Umwandlungsmechanismus eine Taumelplatte (216) beinhaltet.

7. Orthopädisches Impaktwerkzeug (100) nach einem oder einer Kombination von Anspruch 1-6, ferner umfassend einen Dreipositionsauslöseschalter, der eine Aus-Position, eine Einzelbetriebsposition und eine kontinuierliche Betriebsposition beinhaltet.

8. Orthopädisches Impaktwerkzeug (100) nach Anspruch 7, wobei der Dreipositionsauslöseschalter die Einzelbetriebsposition und die kontinuierliche Betriebsposition beinhaltet, die durch zumindest eine Arretierung des Dreipositionsauslöseschalters definiert sind.

9. Orthopädisches Impaktwerkzeug (100) nach einem oder einer Kombination von Anspruch 1-6, ferner umfassend:
einen Energiepegelschalter; und
eine Steuereinheit, die bedienbar ist, um den Motor (270) basierend auf einer Position des Energiepegelschalters zu steuern.

10. Orthopädisches Impaktwerkzeug (100) nach Anspruch 1, ferner umfassend ein Spannfutter, das mit dem Amboss (256) verbunden ist.

11. Orthopädisches Impaktwerkzeug (100) nach Anspruch 1, ferner umfassend eine Rahmenstruktur, die innerhalb des Gehäuses (102) angeordnet ist und die Rohrbaugruppe (230) stützt.

12. Orthopädisches Impaktwerkzeug (100) gemäß Anspruch 11, ferner umfassend eine Taumelplatte, die durch die Rahmenstruktur gestützt ist.

## Revendications

1. Outil à percussion orthopédique (100) comprenant :
un boîtier (102) comprenant une partie de préhension manuelle (104) et une partie boîtier de mécanisme de percussion (112) ;
un ensemble tube (230) comprenant une première partie tube et une seconde partie tube ;
une plaque de percussion inverse (238) disposée entre la première partie tube et la seconde partie tube ;
une enclume (256) comprenant une surface de percussion avant (250) ; et
un mécanisme de percussion (228) comprenant :
un moteur (270),
un piston (236) disposé à l'intérieur de la première partie tube de l'ensemble tube (230), un mécanisme de conversion rotatif-linéaire (216) reliant de manière motrice le moteur (270) au piston (236),
un vérin (260) disposé dans la seconde partie tube et mobile en contact entre la plaque de percussion inverse (238) et la surface de percussion avant (250) ;
ledit ensemble tube (230), ladite enclume (256) et ladite plaque de percussion inverse (238) pouvant être déplacés axialement vers l'arrière à partir d'une position neutre à l'intérieur du boîtier (102) de manière à activer un cycle de percussion avant.

2. Outil à percussion (100) selon la revendication 1, ledit ensemble tube (230), ladite enclume (256) et ladite plaque de percussion inverse (238) pouvant être déplacés axialement vers l'avant depuis une position neutre à l'intérieur du boîtier (102) de manière à activer un cycle de percussion arrière.

3. Outil à percussion orthopédique (100) selon la revendication 1, ledit ensemble tube (230) définissant un orifice de chambre qui est :
ouvert à une atmosphère lorsque l'ensemble tube (230) se trouve dans une position neutre ;
et
fermé à l'atmosphère lorsque l'ensemble tube (230) est déplacé vers l'arrière à partir de la position neutre.

4. Outil à percussion orthopédique (100) selon la revendication 2, ledit déplacement vers l'avant de l'ensemble tube (230) et de l'enclume (256) empêchant le vérin (260) d'entrer en contact avec la surface de percussion avant (250) de l'enclume (256) pendant un cycle de percussion arrière.

5. Outil à percussion orthopédique (100) selon la revendication 1, ledit déplacement vers l'arrière de l'ensemble tube (230) et de la plaque de percussion inverse (238) empêchant le vérin (260) d'entrer en contact avec la plaque de percussion inverse (238) pendant un cycle de percussion avant.

6. Outil à percussion orthopédique (100) selon la revendication 1, ledit mécanisme de conversion rotatif-linéaire comprenant un plateau oscillant (216).

7. Outil à percussion orthopédique (100) selon l'une quelconque ou une combinaison des revendications 1 à 6, comprenant en outre un commutateur de déclenchement à trois positions comprenant une position d'arrêt, une position de fonctionnement unique et une position de fonctionnement continu.

8. Outil à percussion orthopédique (100) selon la revendication 7, ledit commutateur de déclenchement à trois positions comprenant la position de fonctionnement unique et la position de fonctionnement continu définies par au moins un cran du commutateur de déclenchement à trois positions.

9. Outil à percussion orthopédique (100) selon l'une quelconque ou une combinaison des revendications 1 à 6, comprenant en outre :
un commutateur de niveau d'énergie ; et
une unité de commande servant à commander le moteur (270) sur la base d'une position du commutateur de niveau d'énergie.

10. Outil à percussion orthopédique (100) selon la revendication 1, comprenant en outre un mandrin relié à l'enclume (256).

11. Outil à percussion orthopédique (100) selon la revendication 1, comprenant en outre une structure de cadre disposée à l'intérieur du boîtier (102) et supportant l'ensemble tube (230).

12. Outil à percussion orthopédique (100) selon la revendication 11, comprenant en outre un plateau oscillant supporté par la structure de cadre.
